# DEMANDE DE BREVET EUROPEEN

(11) **EP 1 240 889 A1**
(43) Date de publication de la demande: **18.09.2002**
(21) Numéro de dépôt: 02290466.8
(22) Date de dépôt: 26.02.2002
(51) Int. Cl.: A61K 7/06, A61K 7/48

(54) **Composition capillaire épaissie comprenant un polysaccharide greffé par un polysiloxane**

(30) Priorité: 13.03.2001 FR 0103403
(71) Demandeur: L'OREAL, 75008 Paris (FR)
(72) Inventeur: Giroud, Franck, 92110 Clichy (FR); Dupuis, Christine, 75018 Paris (FR)
(74) Mandataire: Bourdeau, Françoise

(57) **Abrégé**

L'invention a pour objet une composition cosmétique capillaire, notamment un gel, comprenant au moins un polymère polysaccharide greffé par un polysiloxane et au moins un épaississant non cellulosique et non fixant. L'invention vise également un procédé cosmétique capillaire comprenant l'application de cette composition sur les cheveux ainsi que son utilisation pour fixer et/ou maintenir la coiffure.

## Description

L'invention a pour objet une composition cosmétique capillaire, notamment un gel, comprenant au moins un polymère polysaccharide greffé par un polysiloxane et au moins un épaississant non cellulosique et non fixant. L'invention vise également un procédé cosmétique capillaire comprenant l'application de cette composition sur les cheveux ainsi que son utilisation pour fixer et/ou maintenir la coiffure.

Au sens de la présente invention, on entend par *« gel »,* une composition initialement liquide, épaissie grâce à un agent ayant des propriétés épaississantes et/ou gélifiantes, ayant in fine une viscosité minimale de 160 cps, de préférence 250 cps à 25°C (viscosimètre Rhéomat 180 - mobile 2 - lecture après 30s).

On entend par « *épaississant non cellulosique »,* tout composé, exempt de motifs cellobiose, capable d'augmenter la viscosité d'une composition cosmétique.

On entend par « *épaississant non fixant »,* tout composé épaississant qui ne suffit pas, à lui seul, lorsqu'il est présent dans une composition cosmétique capillaire, pour fixer et/ou maintenir la coiffure dans une forme voulue.

Il est connu des produits de fixation et/ou de maintien de la coiffure se présentant sous diverses formes, notamment sous forme de gels. Si ceux-ci permettent souvent la fixation durable de la coiffure, ils présentent néanmoins divers inconvénients.

En particulier, de nombreux consommateurs reprochent que le gel reste collé sur les mains lors de l'application, que son temps de séchage sur les cheveux est long, ce qui peut entraîner un effondrement de la coiffure si le gel est appliqué en finition sur cheveux secs, et qu'il donne aux cheveux un aspect artificiel, en particulier une brillance non naturelle.

Il est également reproché aux gels de former une mousse lors de leur application, ce qui nuit à l'esthétique et à la facilité d'application du produit.

Pour résoudre les problêmes ci-dessus, il a déjà été proposé de réaliser des produits cosmétiques capillaires comprenant un polymère fixant et un agent épaississant avec des additifs éventuels. Néanmoins, les compositions connues à ce jour comprenant ces constituants ne donnent pas encore totalement satisfaction, dans la mesure où la coiffure manque encore de naturel et où les gels continuent à trop coller ou mousser sur les mains lors de l'application et à sécher trop lentement.

Le problème posé par l'invention est de fournir une composition cosmétique capillaire, notamment sous forme de gel, qui soit améliorée par rapport aux compositions de l'art antérieur et, en particulier, qui se répartisse de manière homogène, sans donner lieu à la formation d'une mousse lors de son application et qui conduise à des résultats cosmétiques satisfaisants.

De manière surprenante et inattendue, la Demanderesse a découvert qu'en sélectionnant judicieusement le polymère fixant et l'agent épaississant, il est possible de résoudre les problèmes posés ci-dessus.

L'invention a pour objet une composition cosmétique comprenant, dans un milieu cosmétiquement acceptable, au moins un polymère à squelette de type polysaccharide greffé par des chaînons contenant au moins un polysiloxane et au moins un épaississant non cellulosique et non fixant.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition dans une formulation cosmétique capillaire, destinée notamment au maintien et/ou à la mise en forme de la coiffure.

Les polymères à squelettes de type polysaccharide greffés par des chaînons contenant au moins un polysiloxane conformes à la présente invention comprennent une chaîne principale formée à partir de polysacharide(s), sur laquelle se trouve greffé, ainsi qu'éventuellement à l'une au moins de ses extrémités, au moins un chaînon contenant un polysiloxane.

Les polymères à squelettes de type polysaccharide greffés par des chaînons contenant au moins un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs polysaccharide(s), eux-mêmes correctement fonctionnalisés par une fonction, capable de venir réagir avec le ou les groupements fonctionnels portés par ladite silicone pour former une liaison covalente.

Les polymères à squelettes polysaccharide greffés par des chaînons contenant au moins un polysiloxane conformes à l'invention sont choisis, de préférence, parmi ceux décrits dans le brevet US 6 066 727 déposé par la Société Shin-Etsu. Il s'agit de copolymères obtenus par réaction entre un polysaccharide contenant des groupements carboxyles et un polysiloxane contenant un groupement époxy terminal, dans un solvant organique, en présence éventuellement d'un catalyseur.

En particulier, les polymères à squelettes polysaccharide greffés par des chaînons contenant au moins un polysiloxane utilisés plus préférentiellement conformément à la présente invention sont susceptibles d'être obtenus conformément au procédé décrit dans le brevet US 6 066 727, c'est-à-dire en :
(a) mettant en solution, dans un solvant organique, un polysaccharide contenant des groupements carboxyles et un polysiloxane, contenant lui-même un groupement époxy terminal, et répondant à la formule (I) de polysiloxane suivante : 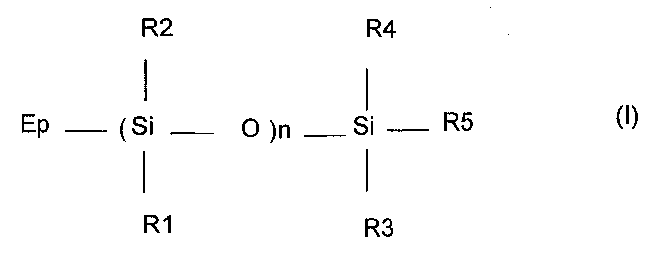 dans laquelle :
   - n est un nombre entier compris entre 3 à 500,
   - R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
   - Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

Parmi des polysaccharides convenant particulièrement pour la mise en oeuvre de l'étape (a) de ce procédé, on peut citer les polysaccharides possédant des groupements carboxylique, benzoyle ou succinoyle, tels que l'hydroxypropyl méthyl cellulose phtalate, l'hydroxypropyl méthyl cellulose acétate succinate, le carboxyméthyl éthyl cellulose, le polysaccharide pullulan acétate phthalate. On préfère, notamment, l'hydroxypropyl méthyl cellulose phthalate et l'hydroxypropyl méthyl cellulose acétate succinate.

De manière avantageuse, R₁, R₂, R₃, R₄ et R₅ sont choisis, indépendamment l'un de l'autre, parmi les groupements méthyl, éthyl, propyl, butyl, cycloalkyl en C₃ à C₈, tels que les radicaux cyclopentyl ou cyclohexyl, aryles, tels que les radicaux phényl et le tolyl, aralkyls en C₃ à C₈, tels que les radicaux benzyl et phénéthyl et alcényl, tels que les groupes vinyl et allyl. Ces radicaux hydrocarbyls monovalents peuvent, éventuellement, être totalement ou partiellement substitués, en particulier, par un atome d'halogène, comme les radicaux chlorométhyl et 3,3,3-trifluoropropyl.

En tant que solvants organiques convenant spécialement bien pour la réalisation de l'étape (a) du procédé, on peut citer les cétones, comme l'acétone ou la cyclohexanone.

L'étape (b) est généralement effectuée sous agitation, éventuellement sous atmosphère inerte.

La composition comprend avantageusement, en pourcentage relatif en poids de la composition, de 0,1 à 20 % de polysaccharides greffés par des polysiloxanes, et plus préférentiellement de 0,5 à 10 % de ce polysaccharide.

La composition comprend avantageusement, en pourcentage relatif en poids de la composition, de 0,01 à 20 % d'épaississant non cellulosique, et de préférence, de 0,05 à 10 %.

De préférence, l'agent épaississant non cellulosique est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv).

Par « épaississant naturel modifié » (i), on entend selon l'invention tout polymère épaississant obtenu par modification chimique simple à partir du polymère naturel lui-même.

On peut citer comme épaississant naturels ou naturels modifiés (i) convenant pour l'invention les gommes de scléroglucane, de gellane, de rhamsan, les alginates, la gomme de karaya, la farine de caroube, les gommes de guar modifiées ou non et leurs dérivés, comme hydroxypropylguar. On utilise notamment la gomme de guar et ses dérivés ( hydroxypropylguar), les gommes d'origine naturelle microbienne (gomme de xanthane, gomme de scléroglucane).

Les copolymères d'acide acrylique et/ou méthacrylique réticulés (ii) sont notamment les copolymères acide acrylique/acrylate d'éthyle et les polymères carboxyvinyliques. De tels polymères sont notamment les "carbomer" (CTFA) vendus par la société GOODRICH sous la dénomination Carbopol.

Les polyacrylamides épaississants réticulés (iii) peuvent plus particulièrement être choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

Les polymères associatifs (iv) de l'invention peuvent être non ioniques, cationiques, anioniques ou amphotères. Ils peuvent, par exemple, appartenir à la classe des polyuréthannes.

Les polyuréthannes associatifs sont des copolymères séquencés comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010 et le Sérad 1035 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS.

Les polymères utilisables dans l'invention sont en particulier ceux décrits dans l'article de G. Fonnum, J. Bakke et Fk. Hansen - Colloid Polym. Sci 271, 380.389 (1993).

Les polyuréthannes associatifs (iv) peuvent particulièrement être choisis parmi :
- les polyéthers polyuréthanes (a) susceptibles d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ et (iii) au moins un diisocyanate et,
- les polyéthers polyuréthanes (b) susceptible d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ différant de celui du polyéther polyuréthane (a) et (iii) au moins un diisocyanate.

De façon préférentielle, on utilisera des polyéthers polyuréthanes (a) et (b) pour lesquels le polyéthylène glycol comporte 150 ou 180 moles d'oxyde d'éthylène.

Toujours de façon préférentielle, on utilisera des polyéthers polyuréthanes (a) et (b) pour lesquels le diisocyanate est le méthylène bis(4-cyclohexylisocyanate).

Une composition plus particulièrement préférée selon l'invention est une composition telle que définie ci-dessus, pour laquelle, le polyéther polyuréthane (a) est obtenu par polycondensation d'au moins trois composés comprenant, un polyéthylèneglycol à 150 ou 180 moles d'oxyde d'éthylène, l'alcool stéarylique (C18) et le méthylène bis(4-cyclohexylisocyanate), et le polyéther polyuréthane (b) est obtenu par polycondensation d'au moins trois composés comprenant, un polyéthylèneglycol à 150 ou 180 moles d'oxyde d'éthylène, l'alcool décylique (C10) et le méthylène bis(4-cyclohexylisocyanate).

Parmi les polyéthers polyuréthanes (a), on peut citer le produit vendu par la société ROHM & HAAS sous la dénomination commerciale :
ACULYN 46, qui est un polycondensat comprenant au moins comme éléments, un polyéthylèneglycol à 150 ou 180 moles d'oxyde d'éthylène, de l'alcool stéarylique et du méthylène bis(4-cyclohexyl-isocyanate) (SMDI), à 15% en poids dans une matrice de maltodextrine (4%) et d'eau (81%).
Parmi les polyéthers polyuréthanes (b), on peut citer le produit vendu par la société ROHM & HAAS sous la dénomination commerciale :
   ACULYN 44, qui est un polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%).

On peut également utiliser, en tant que polyuréthanne associatif, le Viscophobe DB 1000 (Union Carbide).

Comme autres polymères associatifs utilisables, on peut notamment citer les copolymères acide acrylique (Meth) / acrylate d'alkyle C10-C30 comme le Pemulen TR1 de Goodrich.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄, des cétones aliphatiques ou aromatiques, des esters d'acides à chaînes courtes en C₁ à C₈ ou longues et C₉ à C₂₀ et d'alcools à chaînes courtes C₁ à C₈ ou longues C₉ à C₂₀, le pentane, l'heptane, les polyols, les éthers de polyol et l'isodécane.

Parmi les alcools, on peut citer l'éthanol, l'isopropanol. L'éthanol est particulièrement préféré.

Le rapport entre la concentration relative en poids des polymères à squelettes de type polysaccharide et la concentration relative en poids des épaississants non cellulosiques non fixants, dans les compositions conformes à l'invention, est avantageusement compris entre 0,01 et 100, plus avantageusement entre 0,05 et 20, et plus avantageusement encore entre 0,1 et 10.

La composition de l'invention peut également contenir au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'inventions peuvent être appliquées sur la peau, les ongles, les lèvres, les cheveux, les sourcils et les cils.

Les compositions conformes à l'invention sont particulièrement adaptées pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE :

On réalise le gel suivant conforme à la présente invention.

| | |
|---|---|
| CELLULOSE A GREFFONS SILICONE [1] | 4% m.a. |
| SYNTHALEN K [2] | 1.5% m.a |
| 2-AMINO 2-METHYL 1-PROPANOL Q.S.pH | 8 |
| ETHANOL | 35% |
| EAU Q.S.P | 100% |

| | |
|---|---|
| [1] Hydroxy proplyl methyl cellulose acétate succinate à greffons polydiméthylsiloxane, telle qu'elle est synthétisée dans l'exemple N°2 du brevet US 6,066,727 de la société Shin Etsu. | |
| [2] Acide polyacrylique réticulé commercialisé par la Société 3V SA | |

Lors de l'application de ce gel sur la chevelure, aucune formation de mousse n'est observée.

## Revendications

1. Composition cosmétique comprenant, dans un milieu cosmétiquement acceptable, au moins un polymère à squelette de type polysaccharide greffé par des chaînons contenant au moins un polysiloxane et au moins un épaississant non cellulosique et non fixant.

2. Composition selon la revendication 1, **caractérisée par le fait que** les polymères à squelettes de type polysaccharide greffés par des chaînons contenant au moins un polysiloxane comprennent une chaîne principale formée à partir de polysacharide(s), sur laquelle se trouve greffé, ainsi qu'éventuellement à l'une au moins de ses extrémités, au moins un chaînon contenant un polysiloxane.

3. Composition selon l'une quelconque des revendications précédentes, **caractérisée par le fait que** les que les polymères à squelettes de type polysaccharide greffés par des chaînons contenant au moins un polysiloxane sont susceptibles d'être obtenus en :
(a) mettant en solution, dans un solvant organique, un polysaccharide contenant des groupements carboxyles et un polysiloxane, contenant lui-même un groupement 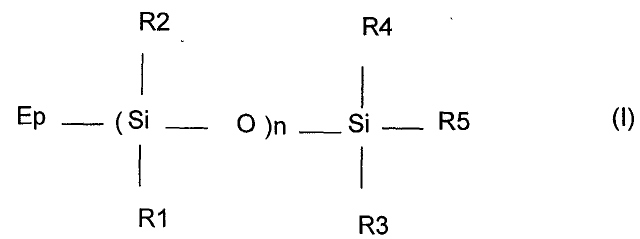 époxy terminal, et répondant à la formule (I) de polysiloxane suivante :
dans laquelle :
- n est un nombre entier compris entre 3 à 500,
- R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
- Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

4. Composition selon la revendication 3, **caractérisée par le fait que** pour la mise en oeuvre de l'étape (a), on utilise, en tant que polysaccharides, des polysaccharides possédant des groupements carboxylique, benzoyle ou succinoyle, tels que l'hydroxypropyl méthyl cellulose phtalalate, l'hydroxypropyl méthyl cellulose acétate succinate, le carboxyméthyl éthyl cellulose, le polysaccharide pullulan acétate phthalate.

5. Composition selon la revendication 3, **caractérisée par le fait que** R₁, R₂, R₃, R₄ et R₅ sont choisis, indépendamment l'un de l'autre, parmi les groupements méthyl, éthyl, propyl, butyl, cycloalkyl en C₃ à C₈, aryls, aralkyls en C₃ à C₈ et alcényl.

6. Composition selon l'une quelconque des revendications précédentes, **caractérisée par le fait que** 1' agent épaississant est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv).

7. Composition selon la revendication 6, **caractérisée par le fait que** le polymère naturel ou naturel modifié (i) est choisi parmi les gommes de xanthane, de scléroglucane, de gellane, de rhamsan, les alginates, la gomme de karaya, la farine de caroube, les gommes de guar et leurs dérivés.

8. Composition selon la revendication 6, **caractérisée par le fait que** les copolymères d'acide acrylique et/ou méthacrylique réticulés (ii) sont choisis parmi les copolymères acide acrylique/acrylate d'éthyle et les polymères carboxyvinyliques.

9. Composition selon la revendication 6, **caractérisée par le fait que** les polyacrylamides épaississants réticulés (iii) sont choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

10. Composition selon la revendication 6, **caractérisée par le fait que** les polymères associatifs (iv) sont des polyuréthannes associatifs.

11. Composition selon l'une quelconque des revendications précédentes, **caractérisée par le fait que** le rapport entre la concentration relative en poids des polymères à squelettes de type polysaccharide et la concentration relative en poids des épaississants non cellulosiques non fixants, dans les compositions conformes à l'invention, est compris entre 0,01 et 100, plus avantageusement entre 0,05 et 20, et plus avantageusement encore entre 0,1 et 10.

12. Composition selon l'une quelconque des revendications précédentes, **caractérisée par le fait que** la composition comprend, en pourcentage relatif en poids de la composition, de 0,1 à 20 % de polysaccharides greffés par des polysiloxanes, et plus préférentiellement de 0,5 à 10 % de ce polysaccharide.

13. Composition selon l'une quelconque des revendications précédentes, **caractérisée par le fait que** la composition comprend, en pourcentage relatif en poids de la composition, de 0,01 à 20 % d'épaississant non cellulosique, et de préférence, de 0,05 à 10%.

14. Composition selon l'une quelconque des revendications précédentes, **caractérisée par le fait qu'**elle contient au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

15. Procédé cosmétique capillaire, en particulier un procédé de fixation et/ou de maintien de la coiffure mettant en oeuvre une composition selon l'une quelconque des revendications 1 à 14.

16. Utilisation d'une composition selon l'une quelconque des revendications 1 à 14 dans une formulation cosmétique capillaire, destinée notamment au maintien et/ou à la mise en forme de la coiffure.
